# EUROPEAN PATENT SPECIFICATION

(11) **EP 3 260 109 B1**
(45) Date of publication and mention of the grant of the patent: **13.05.2020**
(21) Application number: 16768993.4
(22) Date of filing: 15.02.2016
(51) Int. Cl.: A61K 8/02, A61Q 19/00, B65D 83/00

(54) **COSMETIC PRODUCT HAVING IMPREGNATION MEMBER EMBOSSED AND ENGRAVED BY MELTING SURFACE**
KOSMETISCHES PRODUKT MIT DURCH SCHMELZOBERFLÄCHE GEPRÄGTEN UND GRAVIERTEN IMPRÄGNIERELEMENTEN
PRODUIT COSMÉTIQUE COMPORTANT UN ÉLÉMENT D'IMPRÉGNATION GAUFRÉ ET GRAVÉ PAR FUSION DE SA SURFACE

(30) Priority: 23.03.2015 KR 20150040328
(43) Date of publication of application: 27.12.2017
(73) Proprietor: Amorepacific Corporation, Seoul 04542 (KR)
(72) Inventor: JUNG, Sang Young, Seoul 04542 (KR); KIM, Hyeong Sam, Seoul 04542 (KR); CHOI, Jae Young, Seoul 04542 (KR)
(74) Representative: Eder, Michael
(86) International application number: PCT/KR2016/001476
(87) International publication number: WO 2016/153174

(56) References cited:
- JP-A- H09 164 017
- JP-A- 2011 024 754
- KR-B1- 101 355 364
- KR-B1- 101 476 314
- KR-Y1- 200 356 547
- KR-Y1- 200 476 594

## Description

### [Technical Field]

The present invention relates to a cosmetic product having an impregnation member embossed and engraved by melting a surface of the impregnation member. More specifically, the present invention relates to a cosmetic product having an impregnation member embossed and engraved by melting a surface of the impregnation member, wherein the surface of the impregnation member is embossed and engraved by compressing the surface of the impregnation member onto a heated metal mold formed with an emboss-engrave pattern and melting the surface, and the open cell structure of the surface of the impregnation member is melted to convert the same into an open cell structure having a size of 1/2 or less. Thus, when the impregnation member is put with a puff to use the contents, the impregnation member prevents the contents from being wasted by excessively smearing the contents more than necessary, and the contents can be uniformly and thinly smeared on the puff so as to be efficiently used.

Moreover, the present invention relates to a cosmetic product having an impregnation member embossed and engraved by melting a surface of the impregnation member, wherein the impregnation member is embossed and engraved only on an edge portion of the upper surface so as to control the amount of contents discharged through a central portion of the impregnation member and discharged through an edge portion of the impregnation member when the contents are discharged, so that the contents are prevented from flowing out of a cosmetic container.

### [Background Art]

A cosmetic product refers to an article which is used for a human body in order to add the charm of the human body and change the appearance of the human body brighter by making the human body clean and beautiful, or to maintain or promote the health of the skin or hair, while exerting slight effects on the human body.

In general, the cosmetic product is manufactured by mixing mutually different cosmetic materials by using an emulsifying agent such as a surface active agent, and the cosmetic materials may be classified into a water-in-oil (W/O) emulsion type cosmetic material and an oil-in-water (O/W) emulsion type cosmetic material according to the bonding structure between a water-based material and an oil-based material.

The water-in-oil emulsion type cosmetic material, which is obtained by bonding the oil-based material to an outside of the water-based material, has a larger quantity of oil, so that the water-in-oil emulsion type cosmetic material is slowly absorbed into skin and the feeling is heavy upon use, whereas the persistence is longer than that of the oil-in-water emulsion type cosmetic material. Thus, the cosmetic product requiring persistence is manufactured by using the W/O emulsion type cosmetic material to increase water resistance against sweat and water.

In order to compensate the defects of the W/O emulsion type cosmetic material where the feeling is heavy and sticky upon use, the viscosity of content is reduced upon the manufacture of the cosmetic product. However, when the water-in-oil product having low viscosity is stored in a container for a long period of time during distribution, the aqueous material of internal phase and the oil material of external phase may be separated from each other. In this case, it is inconvenient for a user to shake the container to mix the separated aqueous and oil materials with each other for use.

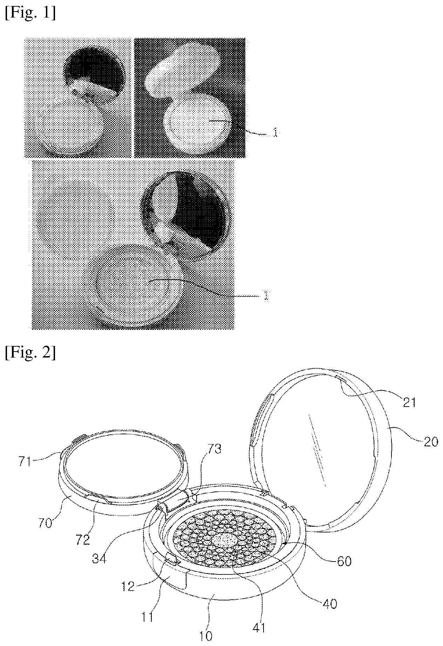

To solve the problems described above, as shown in FIG. 1, a product disclosed in Korean Patent Registration No. 10-1159877 filed by the applicant of the present application has been developed, in which an impregnation member 1 impregnated with water-in-oil contents having low viscosity is mounted in a compact container.

However, according to the above-described related art, since the contents are fully filled in the impregnation member 1 when the cosmetic product is used for the first time, even when the pressure is applied with a small force, the contents are put on the puff more than necessary and wasted.

Further technical background is known from KR 200 476 594 Y1 which refers to a foundation container comprising an impregnated sponge provided with holes.

### [Technical Problem]

An object of the present invention is to provide a cosmetic product for solving the problems described above.

### [Technical Solution]

This is solved by a cosmetic product having the features of claim 1. Accordingly, there is provided a cosmetic product having an impregnation member embossed and engraved by melting a surface of the impregnation member, wherein the surface of the impregnation member has an open cell structure and is embossed and engraved by inserting and compressing the impregnation member in a heated metal mold formed with an emboss-engrave pattern and melting the surface to convert the surface into an open cell structure having a size of ½ or less, and the impregnation member embossed and engraved by melting the surface thereof is impregnated with a cosmetic material.

In addition, a fixing member may be further coupled to an upper end of the inner container to fix the impregnation member such that the impregnation member is prevented from being separated to an outside.

In addition, the surface of the impregnation member may be melted and occluded by the metal mold, and the occluded surface may be perforated by a plurality of needles.

In addition, the impregnation member may be embossed and engraved only on an edge portion of the surface.

Moreover, the impregnation member may be embossed on the surface in a convex circular or polygonal shape.

### [Advantageous Effects]

According to the cosmetic product having the impregnation member embossed and engraved by melting a surface of the impregnation member of the present invention, the surface of the impregnation member is embossed and engraved by compressing the surface of the impregnation member onto a heated metal mold formed with an emboss-engrave pattern and melting the surface, and the open cell structure of the surface of the impregnation member is melted to convert the same into an open cell structure having a size of 1/2 or less. Thus, when the impregnation member is put with a puff to use the contents, the impregnation member can prevent the contents from being wasted by excessively smearing the contents more than necessary, and the contents can be uniformly and thinly smeared on the puff so as to be efficiently used.

In addition, according to the cosmetic product having the impregnation member embossed and engraved by melting a surface of the impregnation member of the present invention, the impregnation member may be embossed on the surface in various sizes of a convex circular or polygonal shape, so that various designs can be implemented to broaden the range of a consumer's choice.

In addition, according to the cosmetic product having the impregnation member embossed and engraved by melting a surface of the impregnation member of the present invention, since the open cell structure of the impregnation member can be partially occluded when the upper surface of the impregnation member is melted with heat so as to be formed with the emboss-engrave pattern, the surface of the impregnation member melted and formed with the emboss-engrave pattern may be pressed with a plurality of needles to allow the occluded surface to be perforated, so that the contents impregnated in the impregnation member can be smoothly discharged.

Moreover, according to the cosmetic product having the impregnation member embossed and engraved by melting a surface of the impregnation member of the present invention, the impregnation member may be embossed and engraved only on an edge portion of the upper surface so as to control the amount of contents discharged through a central portion of the impregnation member and discharged through an edge portion of the impregnation member when the contents are discharged, so that the contents can be prevented from flowing out of a cosmetic container.

### [Description of Drawings]

FIG. 1 is a perspective view showing a compact container according to the related art.
FIG. 2 is a perspective view showing a cosmetic product having an impregnation member embossed and engraved by melting a surface of the impregnation member according to an embodiment of the present invention.
FIG. 3 is an exploded perspective view showing a cosmetic product having an impregnation member embossed and engraved by melting a surface of the impregnation member according to an embodiment of the present invention.
FIG. 4 is a sectional view showing a cosmetic product having an impregnation member embossed and engraved by melting a surface of the impregnation member according to an embodiment of the present invention.
FIG. 5 is a perspective view showing a cosmetic product having an impregnation member embossed and engraved by melting a surface of the impregnation member according to another embodiment of the present invention.
FIG. 6 is a sectional view showing a cosmetic product having an impregnation member embossed and engraved by melting a surface of the impregnation member according to another embodiment of the present invention.
FIG. 7 is a sectional view showing a state of forming an impregnation member accommodated in a cosmetic product having the impregnation member embossed and engraved by melting a surface of the impregnation member according to an embodiment of the present invention.

### [Best Mode]

### [Mode for Invention]

Hereinafter, a cosmetic product having an impregnation member embossed and engraved by melting a surface of the impregnation member according to an embodiment of the present invention will be described with reference to accompanying drawings.

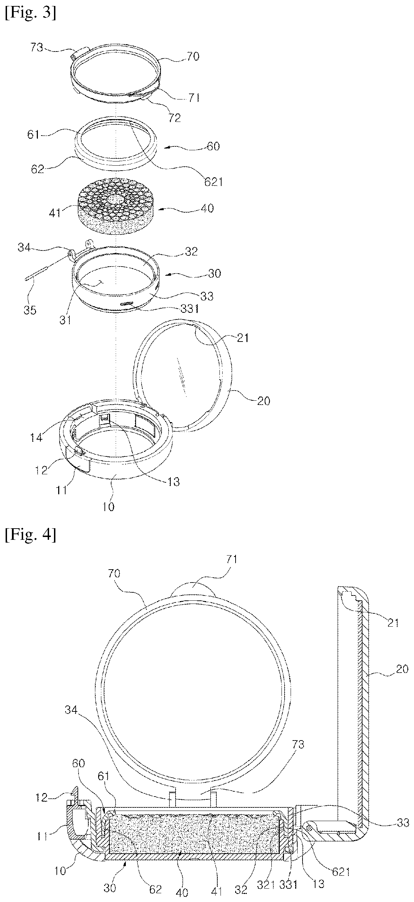

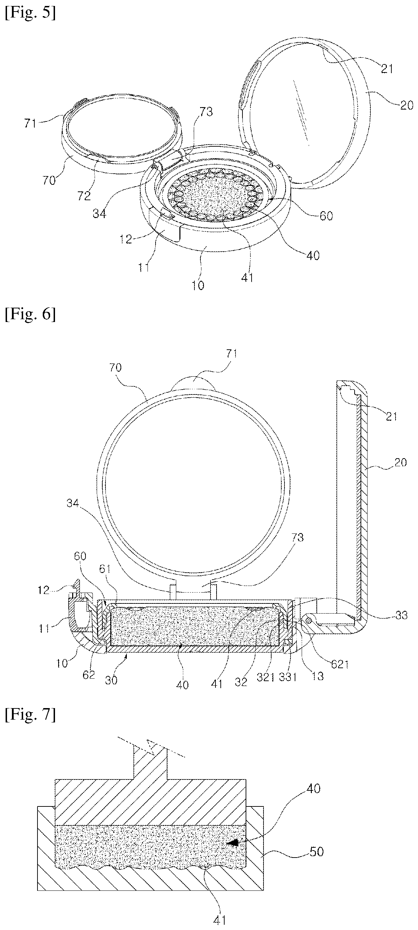

FIG. 2 is a perspective view showing a cosmetic product having an impregnation member embossed and engraved by melting a surface of the impregnation member according to an embodiment of the present invention, FIG. 3 is an exploded perspective view showing a cosmetic product having an impregnation member embossed and engraved by melting a surface of the impregnation member according to an embodiment of the present invention, FIG. 4 is a sectional view showing a cosmetic product having an impregnation member embossed and engraved by melting a surface of the impregnation member according to an embodiment of the present invention, FIG. 5 is a perspective view showing a cosmetic product having an impregnation member embossed and engraved by melting a surface of the impregnation member according to another embodiment of the present invention, FIG. 6 is a sectional view showing a cosmetic product having an impregnation member embossed and engraved by melting a surface of the impregnation member according to another embodiment of the present invention, and FIG. 7 is a sectional view showing a state of forming an impregnation member accommodated in a cosmetic product having the impregnation member embossed and engraved by melting a surface of the impregnation member according to an embodiment of the present invention.

According to a cosmetic product having an impregnation member embossed and engraved by melting a surface of the impregnation member of the present invention, the surface (41) of the impregnation member (40) is embossed and engraved by inserting and compressing the impregnation member (40) in a heated metal mold (50) formed with an emboss-engrave pattern and melting the surface (41), and the impregnation member (40) embossed and engraved by melting the surface (41) thereof is impregnated with a cosmetic material.

The cosmetic material impregnated in the impregnation member (40) may be a gel foundation containing sunscreen agents.

In addition, according to the present invention, the cosmetic product having the impregnation member embossed and engraved by melting the surface of the impregnation member includes: an outer container (10) having an open top; an outer container lid (20) coupled to one side of the outer container (10); an inner container (30) mounted inside the outer container (10); the impregnation member (40) mounted inside the inner container (30) and impregnated with a cosmetic material; and an inner container lid (70) hinged to one side of the inner container (30) so as to be opened and closed, wherein the surface (41) of the impregnation member (40) is embossed and engraved by inserting and compressing the impregnation member (40) in a heated metal mold (50) formed with an emboss-engrave pattern and melting the surface (41).

The outer container (10) is formed on one side surface thereof with a push button (11) having a locking sill (12), has a hinge formed on an opposite side of the push button (11) so as to be hinged to the outer container lid (20), is formed on an inner peripheral surface thereof with a fastening protrusion (13), and is formed on an inner periphery thereof with a hinge bracket mounting groove (14).

The push button (11) allows the locking sill (12) extending from an upper portion of the push button (11) to be easily pushed back by the pushing action of a user, so that the locking sill (12) can be separated from a locking protrusion (21) of the outer container lid (20).

The fastening protrusion (13) is fastened to a fastening groove (331) formed on an outer peripheral surface of an outer wall (33) of the inner container (30).

A hinge bracket (34) of the inner container (30) is inserted into the hinge bracket mounting groove (14).

The outer container lid (20) covers the upper portion of the outer container (10), and is hinged to the outer container (10) so as to be opened or closed.

The locking protrusion (21) is formed on one side of the outer container lid (20), and formed in a protruding shape to correspond to the locking sill (12) of the outer container (10).

The inner container (30) is mounted inside the outer container (10). In addition, the inner container (30) includes a bottom surface (31), an inner wall (32) extending upwards from the bottom surface (31), and an outer wall (33) spaced outwards from the inner wall (32) by a predetermined distance.

The inner wall (32) is formed on an outer peripheral surface thereof with a coupling protrusion (321), and the coupling protrusion (321) is fitted to a coupling groove (621) formed in a fixing member (60) to prevent the fixing member (60) from being separated from the inner container (30).

The outer wall (33) is formed on an outer peripheral surface thereof with a fastening groove (321), and the fastening groove (331) is fastened to the fastening protrusion (13) formed on the inner peripheral surface of the outer container (10) to prevent the inner container (30) from being separated from the outer container (10).

The outer wall (33) is formed on the outer peripheral surface thereof with the hinge bracket (34), and the hinge bracket (34) is hinged with a hinge block (73) formed on the inner container lid (70).

The impregnation member (40) is mounted inside the inner container (30) and impregnated with a cosmetic material, and the impregnation member (40) is formed of at least one material including butadiene rubber (BR), styrene butadiene rubber (SBR), natural rubber (NR), acrylonitrile-butadiene rubber (NBR), wet urethane, dry urethane, polyether, polyester, polyvinyl chloride, polyethylene, latex, silicone, polyvinyl alcohol (PVA), nitrile rubber, butyl rubber, and neoprene.

As shown in FIG. 7, the surface (41) of the impregnation member (40) is embossed and engraved by compressing the surface (41) in the heated metal mold (50) formed with the emboss-engrave pattern and melting the surface (41).

In the impregnation member (40) embossed and engraved on the surface (41) thereof, the open cell structure of the surface (41) is melted to convert the same into an open cell structure having an open cell that has a size of 1/2 or less, so that the cosmetic material can be thinly smeared on a puff so as to be efficiently used.

The impregnation member (40) is embossed on the surface (41) in various sizes of a convex circular or polygonal shape.

Since the open cell structure can be partially occluded when the surface (41) of the impregnation member (40) is melted with heat, the surface (41) is pressed with a plurality of needles to allow the occluded surface (41) to be perforated, so that the cosmetic material impregnated in the impregnation member (50) can be smoothly discharged.

In addition, as shown in FIGS. 5 and 6, according to another embodiment of the present invention, the impregnation member (40) can be embossed and engraved by melting only on the edge portion of the surface (41).

The fixing member (60) is coupled to the inner container (30), and the fixing member (60) includes a horizontal extension piece (61) extending inwards and a downward extension piece (62) extending downwards from the horizontal extension piece (61).

The horizontal extension piece (61) is seated at an upper end of the inner wall (32) of the inner container (30) to prevent the impregnation member (40) from being separated.

The downward extension piece (62) is formed on an inner peripheral surface thereof with the coupling groove (621), and the coupling groove (621) is coupled to the coupling protrusion (321) formed on an outer peripheral surface of the inner wall (32) of the inner container (30) to prevent the fixing member (60) from being separated from the inner container (30).

The inner container lid (70) is coupled to one side of the inner container (30) so as to open and close the inner container (30).

The inner container lid (70) is formed on one side thereof with a handle (71), formed on a lower end thereof with a sealing piece (72), and has the hinge block (73) formed on an opposite side of the handle (71).

The handle (71) makes it easier to open and close the inner container lid (70).

The sealing piece (72) is fitted inside the outer wall (33) of the inner container (30) to improve sealing performance of the inner container (30) when the inner container lid (70) is closely covered on the inner container (30).

The hinge block (73) is fitted to the hinge bracket (34) of the inner container (30), and is fixed by a hinge pin (35).

Hereinafter, a method of assembling and using the cosmetic product having the impregnation member embossed and engraved by melting the surface of the impregnation member according to an embodiment of the present invention will be described in detail.

In order to assemble the cosmetic product having the impregnation member embossed and engraved by melting the surface of the impregnation member of the present invention, the outer container lid (20) is coupled to the outer container (10) on which the push button (11) is formed.

Then, the inner container lid (70) is coupled to the inner container (30).

The impregnation member (40) impregnated with the cosmetic material is mounted in the inner container (30) coupled with the inner container lid (70), in which the surface (41) of the impregnation member (40) is embossed and engraved by compressing the surface (41) in the heated metal mold (50) formed with the emboss-engrave pattern and melting the surface (41) as shown in FIG. 7.

The impregnation member (40) is embossed on the surface (41) in various sizes of a convex circular or polygonal shape.

In addition, as shown in FIGS. 5 and 6, according to another embodiment of the present invention, the impregnation member (40) can be embossed and engraved by melting only on the edge portion of the surface (41).

Thereafter, the fixing member (60) is coupled to the inner container (30) in which the impregnation member (40) is mounted, and the inner container (30) is coupled inside the outer container (10) to complete the assembly.

In the impregnation member (40) of the cosmetic product having the impregnation member embossed and engraved by melting the surface of the impregnation member, which is assembled as described above, the open cell structure of the surface (41) is melted to convert the same into an open cell structure having an open cell that has a size of 1/2 or less, so that a proper amount of the cosmetic material can be uniformly and thinly smeared on the puff, so that the cosmetic material can be efficiently used.

The impregnation member (40) is embossed on the surface (41) in various sizes of a convex circular or polygonal shape, so that various designs can be implemented to broaden the range of a consumer's choice.

As described above, although the cosmetic product having the impregnation member embossed and engraved by melting the surface of the impregnation member according to one embodiment of the present invention has been described for illustrative purposes, the present invention is not limited thereto.

**[Description of Reference numerals]**

| | |
|---|---|
| 10: Outer container | 11: Push button |
| 12: Locking sill | 13: Fastening protrusion |
| 14: Hinge bracket mounting groove | |
| 20: Outer container lid | 21: Locking protrusion |
| 30: Inner container | 31: Bottom surface |
| 32: Inner wall | 321: Coupling protrusion |
| 33: Outer wall | 331: Fastening groove |
| 34: Hinge bracket | 35: Hinge pin |
| 40: Impregnation member | 41: Surface |
| 50: Metal mold | 60: Fixing member |
| 61: Horizontal extension piece | |
| 62: Downward extension piece | |
| 621: Coupling groove | 70: Inner container lid |
| 71: Handle | 72: Sealing piece |
| 73: Hinge block | |

## Claims

1. A cosmetic product comprising an impregnation member embossed and engraved by melting a surface of the impregnation member, wherein the surface (41) of the impregnation member (40) has an open cell structure and is embossed and engraved by inserting and compressing the impregnation member (40) in a heated metal mold (50) formed with an emboss-engrave pattern and melting the surface (41) to convert the surface (41) into an open cell structure having a size of ½ or less, and the impregnation member (40) embossed and engraved by melting the surface (41) thereof is impregnated with a cosmetic material.

2. The cosmetic product of claim 1, the cosmetic product further comprising an inner container (30).

3. The cosmetic product of claim 2, the cosmetic product further comprising:
an outer container (10) having an open top;
an outer container lid (20) coupled to one side of the outer container (10); and
an inner container lid (70) hinged to one side of the inner container (30) so as to be opened and closed,
wherein the impregnation member (40) is mounted inside the inner container (30) and the inner container (30) is mounted inside the outer container (10)

4. The cosmetic product of claim 2 or 3, wherein a fixing member (60) is further coupled to an upper end of the inner container (30) to fix the impregnation member (40) such that the impregnation member (40) is prevented from being separated to an outside.

5. The cosmetic product of one of claims 1 to 3, wherein the surface (41) of the impregnation member (40) is melted and occluded by the metal mold (50), and the occluded surface (41) is perforated by a plurality of needles.

6. The cosmetic product of one of claims 1 to 3, wherein the impregnation member (40) is embossed and engraved only on an edge portion of the surface (41).

7. The cosmetic product of one of claims 1 to 3, wherein the impregnation member (40) is embossed on the surface (41) in a convex circular or polygonal shape.

## Patentansprüche

1. Kosmetisches Produkt, umfassend ein Imprägnierungselement, das durch Schmelzen einer Oberfläche des Imprägnierungselements geprägt und graviert wird, wobei die Oberfläche (41) des Imprägnierungselements (40) eine offene Zellstruktur aufweist und geprägt und graviert wird durch Einführen und Komprimieren des Imprägnierungselements (40) in eine erhitzte Metallform (50), die mit einem Präge-Gravur-Muster ausgebildet ist, und durch Schmelzen der Oberfläche (41), um die Oberfläche (41) in eine offene Zellstruktur mit einer Größe von ½ oder weniger umzuwandeln, und das Imprägnierungselement (40), dessen Oberfläche (41) durch Schmelzen geprägt und graviert wurde, mit einem kosmetischen Material imprägniert ist.

2. Das kosmetische Produkt nach Anspruch 1, wobei das kosmetische Mittel ferner einen Innenbehälter umfasst (30).

3. Das kosmetische Produkt nach Anspruch 2, wobei das kosmetische Produkt ferner umfasst:
einen Außenbehälter (10) mit offenem Oberteil;
einen Außenbehälterdeckel (20), der mit einer Seite des Außenbehälters (10) verbunden ist; und
einen Innenbehälterdeckel (70), der an einer Seite des Innenbehälters (30) so angelenkt ist, dass er geöffnet und geschlossen werden kann,
wobei das Imprägnierungselement (40) im Inneren des Innenbehälters (30) und der Innenbehälter (30) im Inneren des Außenbehälters (10) montiert ist.

4. Das kosmetische Produkt nach Anspruch 2 oder 3, wobei ein Befestigungselement (60) ferner mit einem oberen Ende des Innenbehälters (30) gekoppelt ist, um das Imprägnierungselement (40) so zu befestigen, dass das Imprägnierungselement (40) daran gehindert wird, nach außen hin getrennt zu werden.

5. Das kosmetische Produkt nach einem der Ansprüche 1 bis 3, wobei die Oberfläche (41) des Imprägnierungselements (40) geschmolzen und durch die Metallform (50) verschlossen ist und die verschlossene Oberfläche (41) durch eine Vielzahl von Nadeln perforiert ist.

6. Das kosmetische Produkt nach einem der Ansprüche 1 bis 3, wobei das Imprägnierungselement (40) nur auf einem Randbereich der Oberfläche (41) geprägt und graviert ist.

7. Das kosmetische Produkt nach einem der Ansprüche 1 bis 3, wobei das Imprägnierungselement (40) auf der Oberfläche (41) in einer konvexen kreisförmigen oder polygonalen Form geprägt ist.

## Revendications

1. Produit cosmétique comprenant un élément d'imprégnation gaufré et gravé par fusion d'une surface de l'élément d'imprégnation, dans lequel la surface (41) de l'élément d'imprégnation (40) a une structure à cellules ouvertes et est gaufrée et gravée par insertion et compression de l'élément d'imprégnation (40) dans un moule métallique chauffé (50) formé avec un motif gaufré-gravé et fusion de la surface (41) pour convertir la surface (41) en une structure à cellules ouvertes ayant une taille de ½ ou moins, et l'élément d'imprégnation (40) estampé et gravé par fusion de sa surface (41) est imprégné d'un matériau cosmétique.

2. Le produit cosmétique de la revendication 1, le produit cosmétique comprenant en outre un récipient intérieur (30).

3. Le produit cosmétique de la revendication 2, le produit cosmétique comprenant en outre :
un récipient extérieur (10) à dessus ouvert ;
un couvercle de récipient extérieur (20) couplé à un côté du récipient extérieur (10) ; et
un couvercle de récipient intérieur (70) articulé sur un côté du récipient intérieur (30) de manière à pouvoir être ouvert et fermé,
dans lequel l'élément d'imprégnation (40) est monté à l'intérieur du conteneur interne (30) et le conteneur interne (30) est monté à l'intérieur du conteneur externe (10)

4. Le produit cosmétique selon la revendication 2 ou 3, dans lequel un élément de fixation (60) est en outre couplé à une extrémité supérieure du récipient intérieur (30) pour fixer l'élément d'imprégnation (40) de telle sorte que l'élément d'imprégnation (40) ne puisse pas être séparé de l'extérieur.

5. Le produit cosmétique selon l'une des revendications 1 à 3, dans lequel la surface (41) de l'élément d'imprégnation (40) est fondue et occlusée par le moule métallique (50), et la surface occlusée (41) est perforée par une pluralité d'aiguilles.

6. Le produit cosmétique de l'une des revendications 1 à 3, dans lequel l'élément d'imprégnation (40) est gaufré et gravé uniquement sur une partie marginale de la surface (41).

7. Le produit cosmétique selon l'une des revendications 1 à 3, dans lequel l'élément d'imprégnation (40) est estampé sur la surface (41) selon une forme circulaire ou polygonale convexe.
